# FASCICULE DE BREVET EUROPEEN

(11) **EP 2 591 122 B1**
(45) Date de publication et mention de la délivrance du brevet: **19.02.2020**
(21) Numéro de dépôt: 11743840.8
(22) Date de dépôt: 07.07.2011
(51) Int. Cl.: C12Q 1/00, C12Q 1/04, G01N 33/543, G01N 33/569, B01L 3/00, C12M 1/28, C12M 1/34, C12Q 1/68, G01N 27/26

(54) **PROCEDE DE DETECTION ET D'IDENTIFICATION DIRECTE D'UN MICROORGANISME DANS UN ECHANTILLON BIOLOGIQUE DILUE DANS UN BOUILLON D'ENRICHISSEMENT**
VERFAHREN ZUM DIREKTEN NACHWEIS UND ZUR IDENTIFIZIERUNG EINES MIKROORGANISMUS AUS EINER BIOLOGISCHEN PROBE, DIE MIT EINEM ANGEREICHERTEN KULTURMEDIUM VERDUENNT IST
METHOD FOR THE DIRECT DETECTION AND IDENTIFICATION OF A MICROORGANISM IN A BIOLOGICAL SAMPLE DILUTED IN A SUPPLEMENTED CULTURE MEDIUM

(30) Priorité: 08.07.2010 FR 1055574
(43) Date de publication de la demande: 15.05.2013
(73) Titulaire: Biomérieux, 69280 Marcy L'etoile (FR)
(72) Inventeur: ATRACHE, Vincent, F-69300 Caluire (FR); COLIN, Bruno, F-69280 Marcyl'Etoile (FR); LAFAY, Aurelie, F-69670 Saint Laurent de Vaux (FR); MAKROUF, Bouchra, F-69009 Lyon (FR); MONTES, Pascal, F-69280 Sainte Consorce (FR); MOSTICONE, David, F-69280 Sainte Consorce (FR); RAYMOND, Jean Claude, F-69690 Bessenay (FR); SOFIA, Thierry, F-69480 Marcy (FR); VIMONT, Antoine, F-69005 Lyon (FR)
(86) Numéro de dépôt international: PCT/FR2011/051624
(87) Numéro de publication internationale: WO 2012/004540

(56) Documents cités:
- EP-A2- 0 446 972
- WO-A1-2009/122069
- US-A- 5 795 773
- US-A1- 2009 081 766
- US-A1- 2009 218 239
- VIMONT A. ET AL.: "Growth of Shiga-Toxin producing Escherichia coli (STEC) and bovine feces background microflora in various enrichment protocols", VETERIN. MICROBIOL., vol. 123, no. 1-3, 20 juillet 2007 (2007-07-20), pages 274-281, XP022114804,
- KORRI-YOUSSOUFI H. ET AL.: "Electrochemical biosensing of DNA hybridization by ferrocenyl groups functionalized polypyrrole", ANALYTICA CHIMICA ACTA, vol. 469, no. 1, 26 septembre 2002 (2002-09-26), pages 85-92, XP002604413,
- LE H.Q.A. ET AL.: "Effect of the size of electrode on electrochemical properties of ferrocene-functionalized polypyrrole towards DNA sensing", TALANTA, vol. 81, no. 4-5, 15 juin 2010 (2010-06-15), pages 1250-1257, XP027038797,
- LERMO A. ET AL.: "Towards Q-PCR of pathogenic bacteria with improved electrochemical double-tagged genosensing detection", BIOSENSORS AND BIOELECTRONICS, vol. 23, no. 12, 15 juillet 2008 (2008-07-15), pages 1805-1811, XP022666045,
- ALFONTA L. ET AL.: "Chronopotentiometry and Faradaic impedance spectroscopy as signal transduction methods for the biocatalytic precipitation of an insoluble product on electrode supports: Routes for enzyme sensors, immunosensors and DNA sensors", BIOSENSORS AND BIOELECTRONICS, vol. 16, no. 9-12, décembre 2001 (2001-12) , pages 675-687, XP002604414,
- KIM N. ET AL.: "Application of a flow-type antibody sensor to the detection of Escherichia coli in various foods.", BIOSENSORS AND BIOELECTRONICS, vol. 18, no. 9, 15 août 2003 (2003-08-15), pages 1101-1107, XP002604415,
- YANG L. ET AL.: "Electrical/electrochemical impedance for rapid detection of foodborne pathogenic bacteria", BIOTECHNOLOGY ADVANCES, vol. 26, no. 2, mars 2008 (2008-03), pages 135-150, XP002604416,

## Description

La présente invention concerne, de façon générale, le domaine de l'analyse par exemple l'analyse biologique. Plus spécifiquement, la présente invention concerne un procédé de détection directe et en temps réel d'un microorganisme dans un échantillon dilué ou en suspension dans un bouillon d'enrichissement, à l'intérieur d'un conteneur fermé.

L'analyse microbiologique nécessite des techniques précises et dont le temps d'obtention du résultat doit être le plus court possible.

Dans le domaine médical, il est nécessaire de prévoir et diagnostiquer le risque infectieux : plus le diagnostic est rapide et précis, plus la prise en charge des malades est efficace et le risque de transmission minimisé. L'approche est similaire pour la santé animale.

Dans le domaine agroalimentaire, la problématique est identique. Elle distingue cependant :
- les microorganismes pathogènes et leurs toxines dont la recherche s'applique aux matières premières, produits intermédiaires, produits finis commercialisés,
- les microorganismes non pathogènes, utilisés comme indicateurs-qualité du processus de production, des matières premières aux produits finis, tout au long de la chaîne, et
- les bactéries d'intérêt technologique telles les ferments.

La détection rapide et précise de contaminations présumées permet de les contrôler et d'engager ainsi des actions correctives.

Techniquement, l'analyse microbiologique peut mettre en œuvre une ou plusieurs phases de pré-enrichissement et/ou d'enrichissement, une ou plusieurs phases de détection, une ou plusieurs phases d'énumération des microorganismes. Pour des applications particulières telles le contrôle microbiologique agroalimentaire, une phase de confirmation peut également être requise, afin de répondre aux normes en vigueur dans ce domaine.

A l'heure actuelle, il n'existe pas de méthode pour détecter un microorganisme cible dans une quantité d'échantillon initiale importante, sans faire appel à une étape d'enrichissement.

La phase de pré-enrichissement et/ou d'enrichissement fait appel à des milieux de culture, sélectifs ou non, qui ont pour but de promouvoir la croissance des microorganismes cibles dans les échantillons biologiques ou environnementaux, tout en limitant la croissance des flores non cibles. Les milieux sont souvent utilisés dans des conteneurs du type sac en plastique stérile, dans lesquels ils sont mis en contact avec les échantillons alimentaires ou environnementaux, à fins de remise en suspension et enrichissement des microorganismes recherchés. Cette phase est nécessaire afin de répondre aux besoins qui est de révéler la présence initiale potentielle d'au moins un microorganisme cible dans une quantité d'échantillon très variable et éventuellement très grande, e.g. 25 grammes (g) à 375 g dilué dans 225 à 3375 millilitres (mL) dans le milieu de culture. A l'issue de cette étape d'enrichissement, un aliquote (de 5 microlitres (µl) à 5 mL) est prélevé pour mettre en œuvre l'étape de détection des microorganismes cibles. Or, dans cet aliquote, il est nécessaire de disposer d'une quantité suffisante de microorganismes cibles pour s'assurer de leur détection systématique.

La phase de détection se base historiquement sur la culture des microorganismes sur milieux gélosés, pour la mise en évidence des caractères métaboliques des microorganismes recherchés. On utilise classiquement des substrats enzymatiques spécifiques. Ces substrats enzymatiques sont généralement composés de deux parties, une première partie spécifique de l'activité enzymatique à révéler, appelée également partie cible, et une seconde partie faisant office de marqueur, appelée partie marqueur, généralement constituée par un chromophore ou un fluorophore. Par le choix de ces substrats, selon qu'il y a réaction ou non, il est possible de caractériser la nature d'un microorganisme ou de discriminer différents groupes de microorganismes. Ainsi, l'apparition ou la disparition d'une coloration ou d'une fluorescence sera la signature d'un genre ou d'un type de microorganismes. A cet égard, l'utilisation de milieux chromogènes permet la détection et l'identification simultanées des germes recherchés. Elle simplifie le processus et diminue sensiblement le délai d'obtention du résultat. On citera à titre d'exemple concret les milieux ChromID® de la demanderesse. Ces milieux chromogènes sont basés sur la détection de caractères métaboliques spécifiques des germes recherchés comme par exemple l'activité enzymatique beta-glucuronidase pour *Escherichia coli.*

Les immuno-essais constituent une autre des technologies utilisées pour le test de détection. Ils font appel aux caractéristiques immunogènes des microorganismes recherchés. De façon non exhaustive, on peut citer les techniques ELISA (Enzyme Linked Immuno Sorbent Assay), par compétition ou de type sandwich.

Enfin, les techniques de biologie moléculaire, basées sur les caractères génomiques des microorganismes recherchés, sont également mise en œuvre pour détecter et identifier les microorganismes cibles. On citera à titre d'exemple les techniques d'amplification classiques telles la PCR (Polymerase Chain Reaction) et la NASBA (Nucleic Acid Sequence Based Amplification), qui peuvent être couplées à des techniques de détection en temps réel connues de l'homme du métier.

Toutefois, l'utilisation de toutes ces techniques nécessitent d'ouvrir le sac à l'issue de la phase de pré-enrichissement/enrichissement afin de récupérer un aliquote de l'homogénat et de réaliser l'étape de détection.

La phase de confirmation, quant à elle, est plus particulièrement attachée à l'analyse microbiologique dans le domaine agroalimentaire. En effet, lorsque le résultat des méthodes développées précédemment est positif, il est nécessaire de confirmer la présence du pathogène recherché. Ceci impose un test complémentaire et l'utilisation d'un principe de détection différent de celui utilisé lors de la première analyse. Les techniques décrites *supra* sont à loisir utilisées pour la confirmation.

L'identification complète et précise d'un microorganisme dans un échantillon nécessite donc l'enchaînement de plusieurs étapes : enrichissement, détection et confirmation. La standardisation des tests utilisés en routine a permis l'automatisation des méthodes de détection qui restent cependant longues à mettre en œuvre. Un inconvénient de l'état de la technique est en effet que ces étapes sont réalisées de manière séquentielle et nécessitent un grand nombre de manipulations chronophages, impactant ainsi le temps nécessaire au rendu des résultats.

Par ailleurs, les techniques décrites *supra* nécessitent une ou plusieurs ouverture(s) des sacs d'enrichissement à fins de prélèvement des aliquotes. Or, ceci est d'autant plus dommageable que le nombre d'échantillons négatifs lors du screening (notamment en industries agro-alimentaires) est important. Il est donc intéressant pour le manipulateur de ne pas avoir à ré-ouvrir les conteneurs, pour connaître le résultat de positivité/négativité de l'échantillon considéré.

Eu égard aux problèmes techniques soulevés par l'état de la technique considéré ci-dessus, un des objectifs essentiels de la présente invention est de fournir un procédé simplifié pour la détection, l'identification et la confirmation des microorganismes présents dans des échantillons, en particulier agroalimentaires.

Un autre objectif de la présente invention est de fournir un procédé pour la détection l'identification et la confirmation des microorganismes, qui limite les manipulations de l'échantillon contenu dans le conteneur, limitant de ce fait les risques de contamination, soit du personnel manipulant l'échantillon, soit de l'échantillon lui-même.

Un autre objectif de la présente invention est de fournir un procédé pour la détection et l'identification des microorganismes, qui permet de diminuer le temps nécessaire à l'analyse de l'échantillon.

Un autre objectif de la présente invention est de fournir un procédé pour la détection, l'identification et la confirmation des microorganismes sur le volume total de l'échantillon au cours de l'enrichissement, permettant d'augmenter manifestement la sensibilité, voire la spécificité, de mesure.

Un autre objectif de la présente invention est de fournir un procédé permettant d'augmenter considérablement la cadence d'analyse des échantillons.

Un autre objectif de la présente invention est de fournir un procédé permettant de faire de la multi-détection.

Un autre objectif de la présente invention est d'améliorer la traçabilité de l'analyse du fait de la réduction drastique des étapes de manipulation des échantillons.

Ces objectifs parmi d'autres sont résolus par la présente invention, qui concerne un procédé de détection, d'au moins un microorganisme présent dans un échantillon placé dans un conteneur fermé, ladite méthode comprenant essentiellement les étapes suivantes :
a) Mettre en contact dans le conteneur, ledit échantillon avec (i) au moins un milieu de culture, (ii) un système de révélation apte à permettre la détection, (iii) un support apte à capturer le ou les microorganismes à détecter, ledit support étant constitué de particules magnétiques sur lesquelles est fixé au moins un partenaire de liaison spécifique du ou des microorganismes à détecter, ledit partenaire de liaison spécifique étant choisi parmi les anticorps, les fragments Fab, les fragments Fab', les protéines de phages recombinantes ou non, les phages,
b) Fermer le conteneur,
c) Placer le conteneur dans des conditions aptes à permettre la croissance du ou des microorganisme(s),
d) Appliquer un champ magnétique pour rapprocher les particules magnétiques,
e) Détecter à l'intérieur dudit conteneur fermé, à l'aide d'un moyen de détection, la présence du ou des microorganisme(s) fixé(s) sur le support de capture, la détection étant réalisée en temps réel et mise en évidence par apparition d'une agglutination en temps réel des particules magnétiques.

Par système de révélation, on entend toute molécule capable de se coupler avec les microorganismes ou les partenaires de liaison desdits microorganismes et permettant par leurs propriétés de transduction (fluorescence, coloration, radioactivité notamment) de révéler la présence desdits microorganismes.

Selon un autre mode de réalisation particulier, le procédé selon l'invention comporte une étape intermédiaire c') consistant à transférer tout ou partie du mélange constitué par ledit échantillon, le milieu de culture, le support apte à capturer le ou les microorganismes à détecter et éventuellement un système de révélation, du conteneur, dit alors principal vers au moins un deuxième conteneur dit secondaire, dans lequel il est possible de réaliser éventuellement un enrichissement secondaire, en ajoutant préalablement dans ledit conteneur secondaire, les éléments nutritifs et agents sélectifs *ad hoc.* Un tel enrichissement secondaire permet d'augmenter la population du ou des microorganisme(s) cible(s) par rapport à celle des microorganismes non cibles, ce qui améliore la spécificité.

Avantageusement, le moyen de détection est pris dans le groupe comprenant : les moyens de détection électriques et notamment électrochimiques, les moyens de détection optiques, les moyens de détection acoustiques, les moyens de détection thermiques, les moyens de détection mécaniques, les moyens de détection magnétiques.

Selon un mode particulier de réalisation, il est tout à fait envisageable de coupler les moyens de détection afin de réaliser d'une part la détection et d'autre part, d'effectuer simultanément ou subséquemment la confirmation. Par exemple, il est possible de réaliser la détection du ou des microorganismes cibles au moyen d'un biocapteur électrochimique. Si la fixation des microorganismes cibles est effectuée au moyen de partenaires de liaison spécifiques, l'étape de détection constitue alors une étape d'identification. Une analyse optique des microorganismes fixés spécifiquement sur le biocapteur au niveau de la zone d'analyse, par un dispositif de détection optique permet alors de confirmer l'identification des microorganismes. Si le dispositif de détection optique est un spectromètre Raman, une analyse du spectre Raman par comparaison avec une base de données de spectres de référence correspondants aux différents microorganismes cibles, permet alors de confirmer l'identification dudit microorganisme.

Selon un autre mode de réalisation particulier, il est possible de réaliser la détection et la confirmation avec la même technologie. Ainsi, si le moyen de détection est un moyen optique tel qu'un moyen de mesure de fluorescence intrinsèque, il est particulièrement avantageux de réaliser la détection des microorganismes cibles par apparition de fluorescence intrinsèque. La réponse est alors une réponse oui (présence de fluorescence)/non (absence de fluorescence). Si fluorescence il y a, une analyse spectrale du signal de fluorescence par comparaison avec une base de données de spectres de référence correspondants aux différents microorganismes cibles, permet alors d'identifier ledit microorganisme et par la-même, confirmer la détection de la présence dudit microorganisme.

Selon un mode particulier du procédé selon l'invention, le conteneur est un sac d'homogénéisation. Il peut également s'agir de conteneurs rigides tels que flacons, bouteilles ou piluliers.

Selon un autre mode de réalisation particulier du procédé selon l'invention, le moyen de détection est connecté avec un système d'analyse des données.

De façon avantageuse, la connexion entre le moyen de détection et le dispositif d'analyse des données est une connexion filaire ou une connexion sans fil.

Est également décrit un biocapteur électrochimique pour la détection d'au moins un microorganisme présent dans un échantillon placé dans un conteneur fermé. Ledit biocapteur comprend un support comportant :
- au moins une électrode de détection, revêtue d'au moins un polymère électroactif, sur lequel est fixé par l'une de ses extrémités au moins un oligonucléotide simple brin ou double brin, la deuxième extrémité dudit oligonucléotide étant liée à au moins un partenaire de liaison du ou des microorganismes à détecter, spécifique ou non spécifique ;
- au moins une contre-électrode.

Avantageusement, le polymère électroactif est pris dans le groupe comprenant le polypyrrole, le polyacéthylène, la polyazine, le poly(p-phénylène), le poly(p-phénylène vinylène), le polypyrène, le polythiophène, le polyfurane, le polysélénophène, la polypyridazine, le polycarabazole, la polyaniline.

Selon un mode particulier de réalisation, le polymère électroactif comporte au moins un médiateur électrochimique. Un tel médiateur électrochimique est pris dans le groupe comprenant le ferrocène, la quinone et les dérivés de ceux-ci ou tout autre médiateur bien connu de l'homme du métier.

Selon un mode de réalisation alternatif, le médiateur électrochimique se retrouve sous forme libre dans le milieu de culture. Un tel médiateur peut être par exemple le couple ferricyanure/ferrocyanure[Fe(CN)₆]3⁻/4⁻, le couple chlorure d'iridium [IrCl₆]3⁻/4⁻, le ruthénium hexamine [Ru(NH₃)₆]3⁺/2⁺.

De façon préférentielle, la liaison entre l'oligonucléotide et le partenaire de liaison du ou des microorganismes est réalisé au moyen d'au moins un couple de liaison biotine-streptavidine ou biotine-avidine.

Lorsque l'oligonucléotide est simple brin, une biotine est fixée sur l'extrémité 3' dudit nucléotide, l'extrémité 5' permettant la fixation de ce dernier sur le polymère électroactif, notamment par liaison covalente. En utilisant un partenaire de liaison également biotinylé, il est ensuite aisé de fixer celui-ci à l'extrémité 3' de l'oligonucléotide au moyen d'une molécule de streptavidine ou d'avidine.

Lorsque l'oligonucléotide est double brin, le premier brin est fixé, notamment par liaison covalente au polymère électroactif par son extrémité 5'. Le second brin quant à lui est biotinylé à son extrémité 5', permettant la fixation du partenaire de liaison également biotinylé, au moyen d'une molécule de streptavidine ou d'avidine.

Avantageusement, le partenaire de liaison est pris dans le groupe comprenant : les anticorps, les fragments Fab, les fragments Fab', les protéines de phages recombinantes ou non, les phages.

Les buts et avantages de la présente invention seront mieux compris à la lumière de la description détaillée qui suit, en lien avec le dessin dans lequel :
- La figure 1 représente un sac de pré-enrichissement et/ou d'enrichissement en combinaison avec un biocapteur électrochimique.
- La figure 2 représente une vue de face du biocapteur électrochimique.
- La figure 3A est une représentation de la surface du capteur électrochimique en absence de microorganisme.
- La figure 3B est une représentation de la surface du capteur électrochimique en présence de microorganismes.
- La figure 4 est une représentation schématique d'un système d'analyse du sac pré-enrichissement/enrichissement avec biocapteur électrochimique.
- La figure 5 est un graphique de mesures par spectrométrie d'impédance obtenues lors de la détection d'*E. coli* O157:H7 dans un échantillon alimentaire.
- La figure 6 est un graphique de mesures par spectrométrie d'impédance obtenues lors de la détection de *Listeria innocua* dans un échantillon alimentaire.
- La figure 7 est une représentation schématique d'un système d'analyse par détection optique dans un sac de pré-enrichissement/enrichissement avec support de capture sensibilisé, selon un premier mode de réalisation.
- La figure 8 est une représentation schématique du système d'analyse tel que représenté sur la figure 7, dans une position du sac permettant de lire le support de capture sensibilisé.
- La figure 9 est une représentation schématique d'un support de capture sensibilisé.
- La figure 10 est une représentation schématique du support de capture sensibilisé, représenté sur la figure 8, après analyse dont le résultat est positif.
- La figure 11 est une représentation schématique d'un système d'analyse par détection optique dans un sac de pré-enrichissement/enrichissement avec un support de capture sensibilisé, selon un deuxième mode de réalisation.

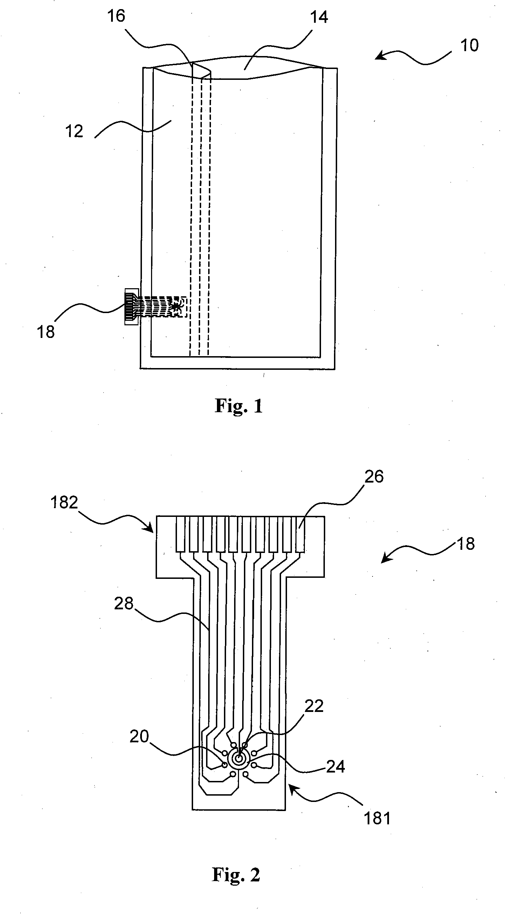

Selon un premier exemple, décrit mais ne faisant pas partie de l'invention, est un procédé de détection, voire d'identification de microorganisme qui consiste à mettre en œuvre un sac d'homogénéisation en plastique et stérile, classiquement appelé sac Stomacher®. Un tel sac est référencé 10 en figure 1. Ce sac 10 est constitué de deux feuilles de plastiques sensiblement rectangulaires, 12 et 14, solidarisées l'une à l'autre par 3 de leurs côtés, de manière à définir un espace intérieur destiné à recevoir le milieu de culture et l'échantillon à analyser. Il comporte accessoirement un filtre 16 de forme sensiblement rectangulaire et relié aux feuilles 12 et 14 par un côté, séparant l'espace intérieur en deux. Le sac comporte enfin un biocapteur 18. Ce biocapteur est un biocapteur électrochimique, représenté en détails en figure 2. Le biocapteur 18 est pris en sandwich entre les feuilles 12 et 14, de sorte qu'une partie 181 correspond à la zone de détection se trouve dans l'espace intérieur du sac 10, alors qu'une partie correspondant à la zone de connexion se trouve à l'extérieur du sac, de manière à permettre la connexion de la puce avec un dispositif d'analyse des données. Ceci est explicité *infra.*

En figure 2, est représenté le biocapteur électrochimique 18 en détails. Comme explicité ci-dessus, le biocapteur 18 est constitué d'une zone d'analyse 181 et d'une zone de connexion. 182. La zone d'analyse comporte huit électrodes de travail 20, disposées autour d'une électrode centrale, appelée contre-électrode, 22. Par ailleurs, la zone d'analyse comporte une électrode de référence 24, sous forme d'un anneau ouvert positionnée autour de la contre-électrode 22. L'ensemble de ces électrodes, sont reliées indépendamment à dix bornes de connexion 26, au moyen de pistes conductrices 28. Les bornes de connexion sont réalisées dans le même matériau que les électrodes de travail. Ce matériau est préférentiellement de l'or. Néanmoins, tout autre matériau conducteur bien connu de l'homme du métier, peut être utilisé, comme le carbone, le platine ou le diamant. Le matériau constituant le support des électrodes est un matériau polymère, tel que du polyimide. Il peut toutefois être envisagé d'utiliser n'importe quel autre matériau présentant des propriétés équivalentes et bien connu de l'homme du métier.

Il est à noter que la configuration d'électrodes présentées en figure 2, n'est qu'une configuration parmi d'autres, et n'est nullement limitative de l'étendue de la protection conférée par la présente demande de brevet.

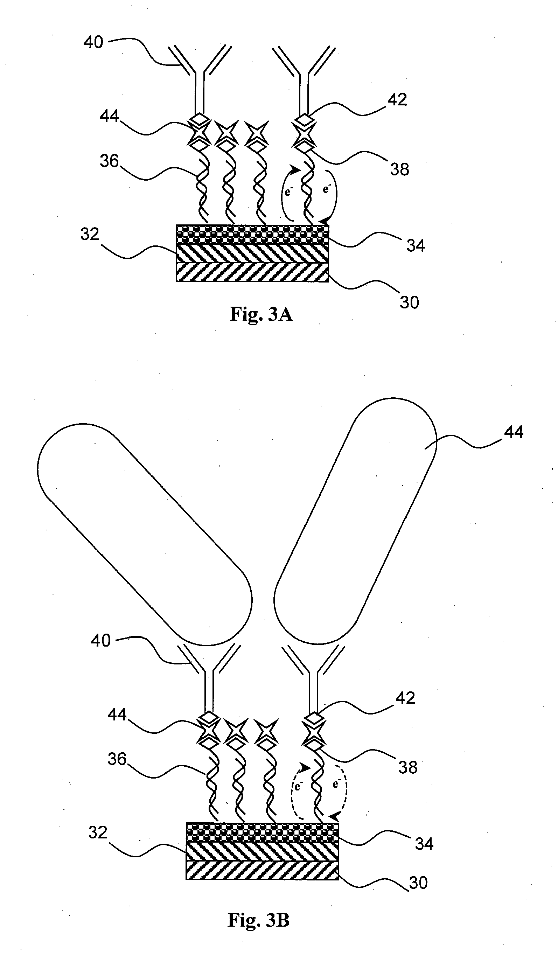

Les figures 3A et 3B représentent une électrode de travail en coupe transversale, au niveau microscopique, respectivement en absence ou présence de microorganismes.

Au niveau des électrodes de travail, on remarque une superposition de trois couches. La première de ces couches 30 est la couche de polymère constituant le support du biocapteur. La couche intermédiaire 32 est la couche de matériau conducteur, typiquement de l'or. Enfin, la couche 34 est une couche de polymère conjugué électro-actif. Un tel polymère est par exemple un polypyrrole. De tels polymères sont bien connus pour leur caractère conducteur et électroactif. Il est également connu que les polypyrroles conservent leur conductivité et leur électroactivité lorsque certains cycles pyrrole sont substitués en position 3 ou 4 avec des groupements fonctionnels. Des polymères portant ce type de groupements fonctionnels sont décrits dans WO-A1 - 95/29199, Garnier et al. (Synthetic Metals, 100: 89-94,1999) Ho-Hoang et al. (Synthetic Metals, 62 : 277-280,1994), Ho-Hoang et al. (J. Mater. Chem., 6 (7), 1107- 1112,1996), et Korri-Youssoufi et al. (Materials Science and Engineering, CI 5, 265- 268,2001). Différentes molécules peuvent ainsi être greffées sur les groupements fonctionnels portés par un monomère de polypyrrole. Ainsi, WO-A1-95/29199 décrit la synthèse d'un polypyrrole obtenu par électro-oxydation à un potentiel supérieur ou égal à 0,8V/ECS. La synthèse du polypyrrole par oxydation électrochimique conduit à la formation d'un film électroactif à la surface de l'électrode, plus précisément sur un substrat conducteur ou sous la forme d'un film auto-supporté. Il s'agit d'une méthode d'immobilisation indirecte des oligonucléotides dans le polypyrrole. Les monomères de pyrroles substitués en position 3 du noyau pyrrole avec des groupements fonctionnels sont dilués dans une solution de monomères non substitués, lesquels immobiliseront les groupements fonctionnels lors de leur électrocopolymérisation par inclusion dans la chaîne des motifs fonctionnels. Dans une deuxième étape, un anti-ligand tel qu'un oligonucléotide, un polynucléotide ou un peptide est couplé chimiquement sur les groupements fonctionnels du polymère précurseur. Le polymère ainsi obtenu conserve ses propriétés conductrices et électroactives. Ces polymères peuvent donc être utilisés pour détecter un analyte interagissant spécifiquement avec l'anti-ligand greffé sur le polymère par la mesure d'une différence de potentiel ou d'une variation de courant: WO-A1-00/77523 décrit également le couplage chimique d'un anti-ligand, tel qu'un oligonucléotide, sur un polymère précurseur portant des groupements fonctionnels.

Il peut également s'agir d'une méthode d'immobilisation directe des oligonucléotides dans le polypyrrole par électrocopolymérisation. Les monomères de pyrroles substitués en position 3 du noyau pyrrole avec des oligonucléotides sont dilués dans une solution de monomères non substitués, lesquels immobiliseront les oligonucléotides directement lors de leur électrocopolymérisation par inclusion dans la chaîne des motifs fonctionnels.

Sur cette couche de polymère électro-actif est greffé un double-brin d'acide nucléique 36, par l'extrémité 5' d'un de ces brins, le brin complémentaire portant une molécule de biotine 38 à son extrémité 5', de sorte qu'une liaison à un partenaire de liaison spécifique 40 lié également à une biotine 42 est possible par le biais d'une molécule de streptavidine 44. Le partenaire de liaison spécifique 40 représentée sur les figures 3A et 3B est un anticorps. Il peut s'agir indifféremment d'un ou plusieurs anticorps monoclonal(aux) ou polyclonaux. Il peut également s'agir d'un fragment d'anticorps, tel qu'un fragment Fab, Fab'2, ainsi que tout anticorps obtenu par modification ou recombinaison génétique, et spécifique d'un microorganisme particulier.

Alternativement, le partenaire de liaison spécifique peut être un phage ou une protéine de phage recombinante, se fixant spécifiquement aux microorganismes cibles. De telles protéines et leur utilisation pour la capture de bactéries ont été décrites entre autres, dans le brevet EP-B-1 356 080.

Il est précisé que la structure représentée aux figures 3A et 3B n'est qu'un exemple parmi d'autres et n'est nullement constitutive d'une limitation de l'invention. En effet, à titre de variante, il peut être envisagé de fixer la molécule anti-ligand directement sur l'électrode sans utiliser le double brin d'acide nucléique.

Afin de réaliser l'analyse, le biocapteur électrochimique, solidaire du sac d'homogénéisation, est mis en contact avec l'échantillon dispersé, le milieu de culture et d'un médiateur électrochimique, tel que le couple ferricyanure - ferrocyanure. En absence de microorganismes, il se produit un échange d'électrons entre le polymère conjugué électro-actif et ledit système redox présent dans le milieu réactionnel. Ceci est matérialisé sur la figure 3A. L'échange électronique est transformé en courant électrique et mesuré par spectroscopie électrochimique, à l'aide d'un potentiostat, tel qu'explicité *infra.*

Lorsque l'échantillon contient des microorganismes 44, ceux-ci sont capturés par les molécules anti-ligands 40. La présence de microorganismes au voisinage de l'électrode, entraîne un encombrement stérique, qui perturbe et diminue le flux électronique entre l'électrode modifiée par le polymère conjugué électro-actif et ledit système redox présent dans le milieu réactionnel. Cette modification est alors mesurée par impédancemétrie et caractérisée par la résistance de transfert de charge, résistance, dont la valeur augmente lorsque la bactérie est capturée (résultat positif).

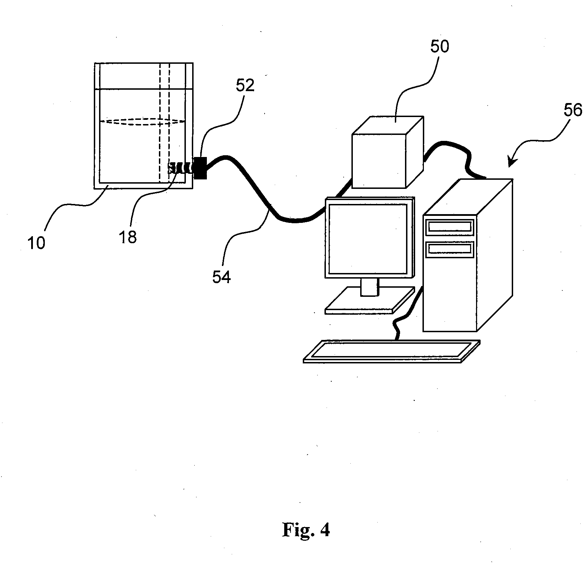

Le système de mesure des résultats d'analyse est représenté schématiquement en figure 4, selon un premier mode de réalisation. Comme on peut le voir sur cette figure, le sac 10, par l'intermédiaire de son biocapteur 18, est connecté à un potentiostat 50. Cette connexion est réalisée grâce à un connecteur 52, connecté à la zone de connexion 182 du biocapteur 18. Le connecteur 52 se prolonge par un câble 54, relié au potentiostat 50. Le potentiostat est, quant à lui, relié à un système informatique 56 apte à enregistrer et analyser les données d'impédancemétrie.

Aux fins de détection des microorganismes, le sac d'homogénéisation 10 est préférentiellement incubé le temps de permettre la croissance desdits microorganismes. Cette incubation peut être classiquement réalisée dans une étuve à une température comprise entre 25 et 45°C. Le temps d'incubation peut varier de 3 à 72 heures en fonction de la quantité initiale de microorganismes présents dans l'échantillon et du type de microorganisme à détecter.

Selon un premier mode de réalisation, la mesure d'impédance peut être réalisée en point final. En effet, le sac d'homogénéisation est incubé le temps considéré comme nécessaire et suffisant à la croissance des microorganismes, puis il est sorti de l'étuve d'incubation et connecté au système de mesure d'impédance décrit supra. La mesure d'impédance est alors réalisée et le résultat est comparé à une valeur d'impédance de référence. Une telle mesure d'impédance est possible dans la mesure où une ou plusieurs électrodes de travail 20 sont recouverte(s) du polymère conjugué électro-actif et/ou d'une molécule anti-ligand non spécifique du microorganisme à détecter. La mesure d'impédance au niveau de cette ou ces électrode(s) constitue la valeur d'impédance de référence. Dans la mesure où la différence entre la valeur d'impédance au niveau des électrodes de détection (électrodes sur lesquelles sont fixées directement ou indirectement les molécules anti-ligands du microorganisme cible) et la valeur d'impédance de référence est supérieure à une valeur seuil, la détection des microorganismes est effective.

Un deuxième mode de réalisation est en pointillés, à savoir par mesures ponctuelles d'impédance au cours de l'incubation. Dans ce cas, le sac d'homogénéisation est sorti de l'étuve d'incubation et connecté au système de mesure d'impédance, pendant le temps nécessaire à ladite mesure, puis remis à incuber. L'intervalle entre deux mesures peut être compris entre 30 secondes à 2 minutes. Ce deuxième mode de réalisation présente comme principal avantage, par rapport au premier mode de réalisation, de permettre de détecter la présence des microorganismes après un temps d'incubation plus court.

Enfin dans un troisième mode de réalisation qui est le mode de réalisation préférentiel, il est prévu de disposer à l'intérieur de l'étuve d'incubation d'un moyen de connexion du biocapteur au système de mesure d'impédance. Il peut s'agir d'un système de connexion filaire ou sans fil. Un tel mode de réalisation est particulièrement avantageux car il permet de réaliser une mesure à intervalle régulier à l'intérieur des sacs d'homogénéisation sans avoir à manipuler ces derniers. Par ailleurs, la mesure à intervalle régulier d'impédance permet de réaliser une détection en temps réel des microorganismes. Associé à un système d'alerte informatique du personnel technique lorsqu'une détection est réalisée, ce dernier n'est alors plus contraint par le flux de travail consistant à faire des mesures successives dans le temps. Il doit alors uniquement intervenir quand un microorganisme est détecté dans un sac.

Un moyen de connexion filaire est constitué par tout moyen permettant de connecter deux dispositifs électroniques entre eux, afin de permettre la transmission de données. En particulier, un moyen de connexion filaire peut être un système de connexion de type série (norme RS 485, RS 232), de type USB (Universal Serial Bus), de type réseau (Ethernet), de type parallèle (GPIB) ou tout autre moyen équivalent.

Un moyen de connexion sans fil est un dispositif d'émission - réception d'ondes radioélectriques. Il peut s'agir par exemple d'un système Wifi (norme 802.11b), Bluetooth (norme 802.15) ou encore ZigBee (norme 802.15.4).

Selon une alternative, le moyen d'acquisition de données peut être un lecteur RFID (Radio Frequency Identification), une carte Labjack ou tout autre moyen bien connu de l'homme du métier.

Selon un deuxième exemple, décrit mais ne faisant pas partie de l'invention, le procédé peut être mis en œuvre grâce à un moyen de détection optique. Ce moyen de détection peut être indépendant du support de capture. C'est le cas par exemple d'un capteur optique, tel une caméra. Alternativement, le moyen de détection optique et le support de capture peuvent ne faire qu'un. C'est le cas par exemple d'une fibre optique dont l'extrémité joue le rôle de support de capture.

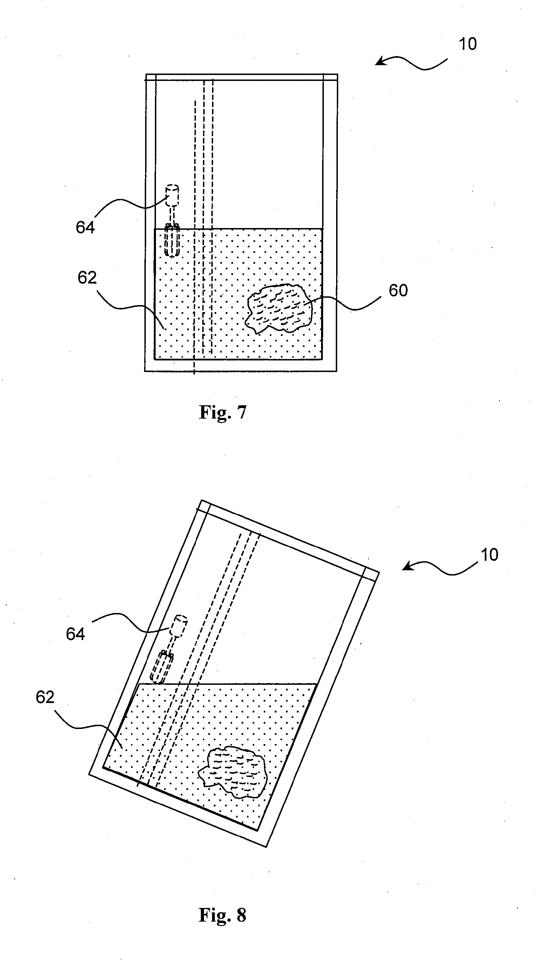

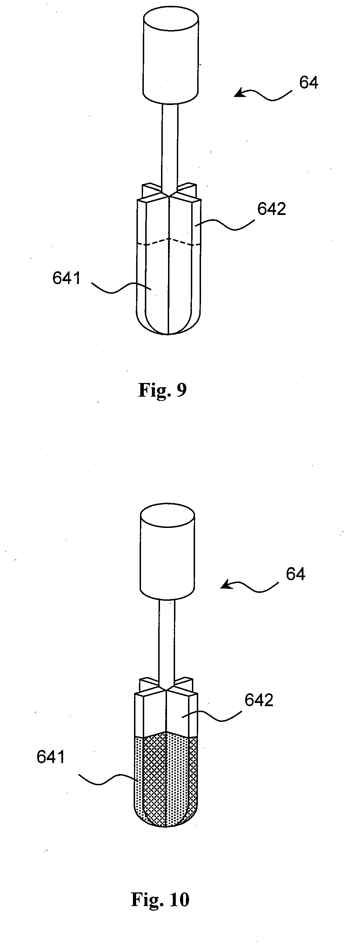

Une telle alternative est représentée sur la figure 7. Un sac d'homogénéisation 10 fermé, tel que décrit précédemment, est incubé avec un échantillon alimentaire 60, constitué ici par un échantillon de steak haché. Cet échantillon alimentaire 60 est plongé dans un milieu de culture 62, implémenté avec un système de révélation. Un support de capture sensibilisé 64, maintenu en place dans le sac par tout moyen approprié, est également placé dans le sac d'homogénéisation 10 et est immergé dans le milieu de culture 62. Le support de capture sensibilisé 64 est fonctionnalisé par au moins un partenaire de liaison spécifique d'un microorganisme cible à détecter. Le support de capture peut être constitué de tout support apte à permettre la fixation de partenaires de liaison spécifique et bien connu de l'homme du métier. A titre d'exemple non limitatif, un support de capture adéquate peut être en polystyrène irradié, tel que celui commercialisé par la société Nunc/Thermo Scientific (Cat. No. 472230). Un tel support de capture est représenté schématiquement sur la figure 9, sous la référence 64. La partie inférieur peut être avantageusement et selon un mode de réalisation préférentiel, divisée en deux. La zone référencée 641 peut être sensibilisée avec une solution de partenaires de liaison (anticorps polyclonaux, anticorps monoclonaux, fragments Fab' ou Fab'2, protéines de phage), alors que la partie supérieure 642 reste-elle vierge de tout partenaire de liaison et joue ainsi un rôle de contrôle négatif.

Le support de capture est fonctionnalisé par au moins un partenaire de liaison spécifique tel qu'anticorps, aptamères, phages, protéines de phage recombinant, ou tout moyen équivalent, permettant la capture spécifique des bactéries cibles.

Ces dernières peuvent être colorées simultanément à leur croissance grâce au système de révélation contenu dans le milieu de culture.

Selon un exemple particulier, le système de révélation est basé sur la réduction du TTC par les microorganismes. Simultanément à la croissance, le TTC (incolore sous sa forme non réduite) est internalisé par lesdits microorganismes, puis réduit par ces derniers en triphényl-formazan (rouge) colorant ainsi lesdits microorganismes en rouge et permettant alors leur révélation sur le support.

Le procédé de détection directe et en temps réel de microorganismes dans un échantillon alimentaire, pendant la période d'incubation, est effectué par lecture optique d'un support de capture sensibilisé, de manière automatisée ou non. L'incubation peut être réalisée à des températures comprises entre 25 et 44°C pendant 6 à 48h.

Aussi une fois la capture effective d'une certaine quantité de microorganismes cibles colorés (cas d'un échantillon positif), il se produit un changement des propriétés optiques du support par apparition d'une coloration rouge sur celui-ci (i.e. transduction du signal biologique). Cette coloration du support de capture est alors détectable à l'œil ou mesurable via l'utilisation d'un automate de lecture tel qu'une caméra. Le support de capture est représenté schématiquement sur la figure 10 après analyse dont le résultat est positif. Comme on peut le voir, la zone 641 apparaît colorée du fait de la fixation des microorganismes cibles sur les partenaires de liaison spécifiques. La zone 642 jouant le rôle de témoin négatif demeure quant à elle, de la couleur initiale du support de capture.

Pour faciliter la lecture, il est préférable que le support de capture sensibilisé ne soit plus en contact avec le milieu de culture. A cette fin, il peut être envisagé par exemple de pencher le sac d'homogénéisation 10, ceci est bien représenté sur la figure 8. Comme explicité *supra,* la lecture peut être faite en point final, en pointillés ou en temps réel.

Selon le procédé selon l'invention, le support de capture est constitué par des particules sensibilisées, à savoir portant un partenaire de liaison spécifique ou non spécifique du ou des microorganisme(s) à détecter. La détection est alors préférentiellement mise en évidence par apparition d'une agglutination en temps réelles des particules sensibilisées, par l'intermédiaire des microorganismes cibles liés à ces dernières, au cours de la période d'incubation. Un tel mode de réalisation est décrit dans le document WO-A-2009/122069.

Selon un mode de réalisation particulier, les particules sensibilisées peuvent être des particules magnétiques. Ce mode de réalisation consiste a détecter directement, via l'agglutination de particules magnétiques sensibilisées, la présence du microorganisme cible (e.i. *E. coli* O157:H7) dans un échantillon alimentaire en cours d'enrichissement. La détection s'effectue pendant la période d'incubation en immergeant les particules magnétiques sensibilisées avec un partenaire de liaison spécifique (i.e. protéine de phage recombinante anti-*E*. *coli* O157:H7) dans le conteneur fermé qui contient l'échantillon alimentaire, dilué dans le milieu de culture.

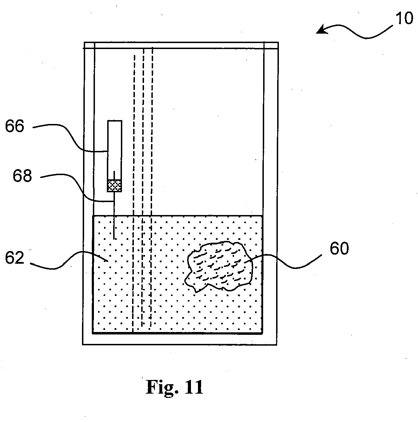

Dans cette alternative, il peut être avantageux d'utiliser un conteneur secondaire, du type tube, à l'intérieur du conteneur principal (sac d'homogénéisation) afin d'améliorer la mise en évidence de l'agglutination de particules sensibilisées. Comme on peut le voir sur la figure 11, le sac d'homogénéisation 10 contient en plus du milieu de culture 62 et de l'échantillon 60, un tube 66. Ce tube 66 est en communication fluidique avec le milieu de culture 62 contenu dans le sac d'homogénéisation 10 par le biais d'un conduit 68. Une fraction du milieu de culture 62 contenant l'échantillon alimentaire peut alors être transféré dans le tube 66, dans lequel se fait la détection. Un tel transfert peut notamment être réalisé par changements de température, basés sur la loi des gaz parfaits (PV=nRT). Un tel procédé est décrit dans le document WO-A-2004/092401.

Dû à l'utilisation de particules magnétiques, la lecture dans le conteneur secondaire contenant le milieu réactionnel à l'issue de la période d'incubation se fera à l'aide d'un lecteur magnétique.

Le signal magnétique peut être amplifié grâce à l'utilisation préalable d'un champ magnétique (via un aimant) qui concentre l'agglutination au centre de la zone de lecture.

L'application d'un champ magnétique peut également améliorer la limite de détection, lorsque ce phénomène provoque la formation d'une agglutination, suite au rapprochement des particules magnétiques ayant capturé les microorganismes. En effet, si la concentration en microorganismes est insuffisante pour provoquer une agglutination passive, le fait de rapprocher entres elles les particules magnétiques, dont certaines auront au préalable capturées les microorganismes, va forcer la formation d'une agglutination. De plus, la répétition de cette séquence (i.e. aimantation et remise en suspension) peut également amplifier le phénomène de capture et de formation d'une agglutination et donc amplifier la sensibilité de l'analyse.

Les exemples présentés ci-après ont pour but de présenter différents modes de réalisation du procédé selon l'invention et les résultats obtenus. Ils ne sont nullement limitatifs de l'invention.

### EXEMPLES

### Exemple 1: Préparation des électrodes d'analyse du biocapteur électrochimique

### Réactifs :

Le perchlorate de lithium (LiClO₄), le chlorure de sodium (NaCl), l'hydroxyde de sodium (NaOH), l'hexacyanoferrate de potassium (III) (K₃Fe (CN)₆), l'hexacyanoferrate de potassium (II) trihydraté (K₄Fe(CN)₆; 3H₂O), le Tween 20, le tampon phosphate (BPS), le sérum albumine bovine (BSA), le tris(hydroxymethyl)aminométhane (TRIS), l'acide maléique, l'ADN de Saumon et le Denhardt 50X proviennent de la société Sigma-Aldrich.

Le tampon de lavage pH 7.2 est du PBS 0,01M, NaCl 0,5M et 0,05 % Tween.

Le tampon d'hybridation est du PBS 0,01M, NaCl 0,5M, Denhardt 2X et l'ADN de Saumon à 10 µg/mL.

Le tampon de greffage du partenaire de liaison est le tampon TRIS-MALEATE BSA pH 6.2 constitué de TRIS 24,23 g/L, d'acide maléique 23,2 g/L, d'hydroxyde de sodium 6 g/L et de BSA 5 g/L.

Le 3-(2-hydroxyethyl)pyrrole ou PyOH et le 3-(phtalimide ethanoate)pyrrole ou PyNHP sont fournis par EZUS Lyon.

Des oligonucléotides synthétiques comportant 20 nucléotides et portant un groupe amino à l'extrémité 5' sont fixés de manière covalente par substitution des groupes NHP.

Le monomère fonctionnel est le suivant : Pyr-^{5'}TTTTTTTTTTGAATCCTCAGTTTTTCAACG^{3'}.

L'oligonucléotide complémentaire porte un groupe biotine à l'extrémité 5'. Sa séquence est la suivante : ^{5'}CGTTGAAAAACTGAGGATTC^{3'}.

### Biocapteur et matériel de détection électrochimique :

Les mesures de détection électrochimique sont réalisées avec un potentiostat BioLogic de Sciences Instruments, contrôlé par ordinateur.

Le capteur utilisé est issu de la technologie des circuits imprimés (PCB). Le dépôt d'or sur les électrodes est un dépôt galvanique par électrolyse à partir d'un bain à base d'or. Les électrodes sont composées d'une multicouche époxy, cuivre, nickel et or.

### Préparation des électrodes :

Aux fins de lavage des électrodes, la zone d'analyse des capteurs est trempée dans une solution eau distillée/éthanol 1:1, pendant une minute dans un bain à ultrasons.

Après lavage, les capteurs sont nettoyés et activés électrochimiquement. Pour ce faire, une goutte de 30 µL de NaOH 0.2 M dans l'eau distillée est déposée sur la zone d'analyse du capteur, de manière à mouiller l'intégralité des électrodes. Le capteur est connecté à un potentiostat et plusieurs cycles de saut de potentiel en oxydation et en réduction sont imposés par chronoampérométrie. Le but de cette étape est de générer des bulles d'oxygène à l'interface avec les électrodes de manière à supprimer tout contaminant organique et/ou inorganique. Le capteur est ensuite rincé avec de l'eau distillée.

La surface des électrodes de travail est modifiée par électrodéposition de copolymère. Ainsi toutes les électrodes sont recouvertes d'une goutte de solution d'électropolymérisation 100 mM de PyOH et 25 µM de PyODN (rapport de concentration 1/4000) et 0.5 M LiCLO₄. La réaction est ensuite électroconduite par application d'un potentiel fixe de 0.8V/pseudo référence or, imposé par chronoampérométrie. La polymérisation est interrompue une fois que la charge imposée de 11 mC/cm² est atteinte. Le copolymère est formé simultanément sur toutes les électrodes de travail. Les électrodes sont rincées ensuite à l'eau distillé.

L'étape suivante consiste dans l'hybridation. Le capteur est recouvert avec une goutte de 30 µL de solution tampon en présence de 100 nM d'ODN cible biotinylé. L'hybridation est réalisée à 37 °C pendant 30 minutes. Après une étape de lavage au tampon PBS, le capteur est trempé dans une solution de streptavidine 100 µg/mL dans le tampon PBS pendant 15 minutes avec agitation. La molécule anti-ligand est ensuite fixée en mettant le capteur en contact avec une solution de molécule anti-ligand à 1 µg/mL dans le tampon TRIS-maléate BSA.

Dans les exemples suivants, la molécule anti-ligand est soit une protéine recombinante de phage pour la détection *d'E.coli* O157 ou un fragment Fab' pour la détection de *Listeria spp.*

### Exemple 2: Détection d'E. coli O157 :H7 dans un échantillon alimentaire

Un biocapteur fonctionnalisé avec des protéines recombinantes de phage spécifique d'*E .coli* O157, tel que décrit *supra* et solidarisé à un sac d'homogénéisation est incubé avec un échantillon alimentaire.

Deux sacs comportant les biocapteurs sont incubés avec des préparations d'enrichissements positifs. Deux sacs comportant les biocapteurs sont incubés avec les préparations d'enrichissements négatifs et deux sacs comportant les biocapteurs sont incubés avec une préparation d'enrichissement non contaminée de manière à mesurer le bruit de fond de la matrice alimentaire.

### Préparation d'enrichissements positifs

25 g de viande crue ayant au minimum 5 % de matières grasses sont placés de manière aseptique dans le sac Stomacher® ® avec filtre et mis en contact avec *E.coli* O157:H7 ATCC 43888. Le sac est placé 24 heures à 2-8°C pour stresser la souche.

Ensuite, 225 mL d'eau peptonée tamponnée (ref 42043 bioMérieux) préchauffés 24 heures à 41,5°C et 5 mM de sonde redox [Fe(CN)6]3⁻/4⁻ sont ajoutés à l'échantillon.

La croissance des bactéries *E.coli* O157 :H7 en présence de 5 mM de sonde redox [Fe(CN)6]³⁻/⁴⁻ a été préalablement vérifiée. Il a été confirmé que la présence de cette sonde redox ne ralentissait pas la croissance bactérienne au sein d'un milieu de culture.

Après homogénéisation de la suspension, un support de capture fonctionnalisé est placé dans le sac Stomacher® ®.

Ce protocole est répété afin de tester deux suspensions positives, au moyen de deux sacs comportant un support fonctionnalisé.

Un dénombrement sur boite de Pétri, à partir des deux suspensions positives a permis d'évaluer, avant incubation dans le sac Stomacher® ®, une concentration moyenne en *E.coli* O157:H7 ATCC 43888 à 0,92 UFC/g de viande crue.

La préparation est alors incubée à 41,5°C pendant 3 heures.

### Préparation des enrichissements négatifs

Contrôle négatif : à partir du même numéro de lot de viande crue, 25 g sont placés de manière aseptique dans un sac Stomacher® ® avec filtre et mis en contact avec *Bacillus cereus* ATCC 27522 . Le sac est placé 24 heures à 2-8°C pour stresser la souche.

Ensuite, 225 mL d'eau peptonée tamponnée (ref 42043 bioMérieux) préchauffés 24 heures à 41,5°C et 5 mM de sonde redox [Fe(CN)6]³⁻/⁴⁻ sont ajoutés à l'échantillon.

Après homogénéisation de la suspension, un support de capture fonctionnalisé est placé dans le sac.

Ce protocole est répété afin de tester deux suspensions négatives, au moyen de deux sacs comportant un support fonctionnalisé.

Dans les deux suspensions, le taux de contamination par *Bacillus cereus* ATCC 27522, avant incubation dans le sac Stomacher® ®, est évalué à 1,32 UFC/g de viande crue (mesure théorique).

La préparation est alors incubée à 41,5°C pendant 3 heures.

Mesure du bruit de fond généré par la matrice : le même protocole sans bactéries, est répété. La préparation est incubée à 41,5°C pendant 6 heures.

Le but de cet essai est de vérifier s'il est possible de détecter *E. coli* O157 :H7 après 3 heures d'incubation / enrichissement.

### Résultats obtenus:

Pour chaque sac, la mesure d'impédance est réalisée directement, sans étape de lavage du biocapteur.

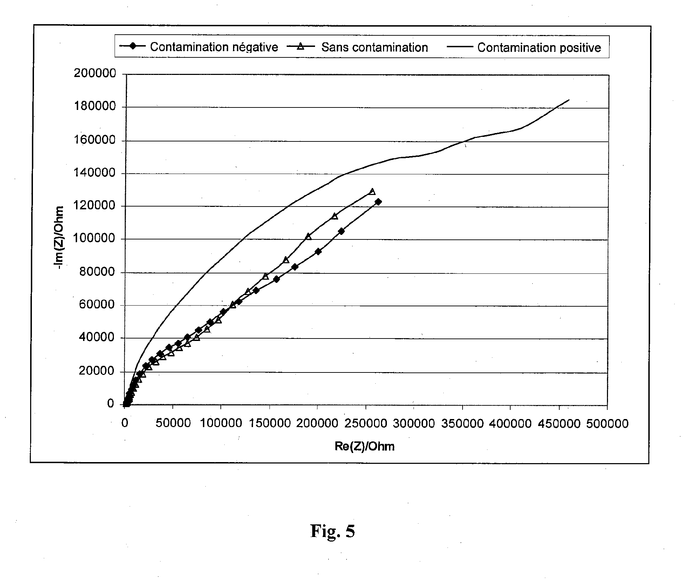

Les graphes Nyquist (-Im(z) vs. Re(z)) obtenus à 200 mV dans une échelle de fréquence comprise entre 1 Hz to 100 kHz sont regroupés sur la figure 5.

Le spectre d'impédance électrochimique obtenu après 6 heures d'incubation de la préparation de viande crue dans le sac Stomacher® sans contamination et celui obtenu après 3 heures d'incubation de la préparation de viande crue avec contamination négative montrent la même résistance de transfert d'électrons R_{ct} (diamètre des demi-cercles), les valeurs sont respectivement égales à 116 et 115 kΩ.

La résistance au transfert d'électrons est donc attribuée au bruit de fond de la matrice, qui est identique à la résistance au transfert d'électrons à la croissance des bactéries non cibles.

Après 3 heures d'enrichissement de la préparation de viande crue avec contamination positive, par *E. coli* O157:H7 (0,92 UFC/g de viande crue), la valeur de résistance au transfert d'électron est de 719 kΩ, soit environ six fois supérieure à celle obtenue avec la contamination négative et permet donc une détection nette de *E. coli* O157:H7.

La valeur R_{ct} moyenne obtenue avec l'intégralité des sacs d'homogénéisation, les valeurs de déviation standard et de coefficient de variation, sont regroupées dans le tableau 1, ci dessous. Les valeurs indiquées sont les valeurs brutes de résistance de charge correspondant au diamètre du demi cercle du signal d'impédance.

**Tableau 1**

| | Bruit de fond matrice (sans contamination) | Contamination negative *B.cereus* (1,32 CFU/g) | Contamination positive *E. coli* (0,92 UFC/g) |
|---|---|---|---|
| Moyenne R_{ct} (kΩ) | 109 | 90 | 708 |
| Déviation standard | 9 | 8 | 25 |
| Coefficient variation (%) | 8 | 9 | 4 |
| Nombre de mesures | 16 | 7 | 16 |

### Exemple 3 : Détection de Listeria innocua dans un échantillon alimentaire

Un biocapteur fonctionnalisé avec des fragments Fab' spécifiques de *Listeria,* tel que décrit *supra* et solidarisé à un sac d'homogénéisation est incubé avec un échantillon alimentaire.

Un sac comportant les biocapteurs est incubé avec une préparation d'enrichissement positif. Un sac comportant les biocapteurs est incubé avec une préparation d'enrichissement négatif.

### Préparation de l'enrichissement positif

25 g de viande crue ayant au minimum 5 % de matières grasses sont placés de manière aseptique dans un sac Stomacher® avec filtre et mis en contact avec *Listeria innocua* ATCC 33090. Le sac est placé 22 heures à 2-8°C pour stresser la bactérie.

Ensuite, 225 mL bouillon *Listeria* XPress (ref. 42626 bioMérieux) préchauffés 18 heures à 30°C et 5 mM de sonde redox [Fe(CN)6]3⁻/4⁻ sont ajoutés à l'échantillon.

La croissance des bactéries *Listeria innocua* en présence de 5 mM de sonde redox [Fe(CN)6]3⁻/4⁻ a été préalablement vérifiée. Il a ainsi été confirmé que la présence de cette sonde redox n'inhibait pas la croissance bactérienne au sein d'un milieu de culture.

Après homogénéisation de la suspension, deux supports de capture fonctionnalisés sont placés dans le sac Stomacher® .

Un dénombrement sur boite de Pétri a permis d'évaluer la concentration en *Listeria innocua* ATCC 33090, avant incubation dans le sac Stomacher®, à 0,48 UFC/g de viande crue.

La préparation est alors incubée à 30°C pendant 6 heures.

### Préparation de l'enrichissement négatif

Contrôle négatif : à partir du même numéro de lot de viande crue, 25 g sont placées de manière aseptique dans un sac Stomacher® avec filtre et mis en contact avec *Staphylococcus aureus* ATCC 6538P. Le sac est placé 22 heures à 2-8°C.

Ensuite, 225 mL bouillon *Listeria* XPress (ref. 42626 bioMérieux) préchauffés 18 heures à 30°C et 5 mM de sonde redox [Fe(CN)6]3⁻/4⁻ sont ajoutés à l'échantillon.

Après homogénéisation de la suspension, deux supports de capture sont placés dans le sac Stomacher® . La concentration en *Staphylococcus aureus* ATCC 6538P est de 4,10⁷ UFC/g de viande crue (mesure théorique), avant incubation dans le sac Stomacher® .

La préparation est alors incubée à 30°C pendant 6 heures.

Le but de cet essai est de vérifier s'il est possible de détecter les bactéries du genre *Listeria* après 6 heures d'incubation/enrichissement.

### Résultats obtenus :

Pour chaque sac, la mesure d'impédance est réalisée directement, sans étape de lavage du biocapteur.

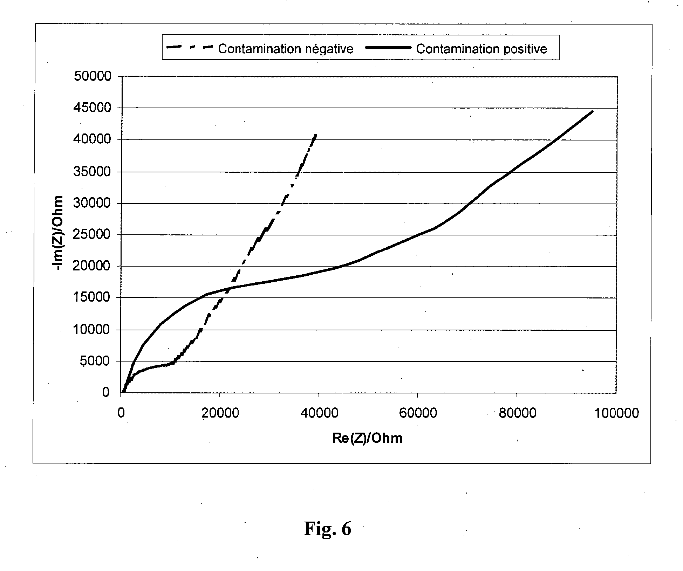

Les graphes Nyquist (-Im(z) vs. Re(z)) obtenus à 200 mV dans une échelle de fréquence comprise entre 1 Hz to 100 kHz sont regroupés sur la figure 6.

Le spectre d'impédance électrochimique obtenu après 6 heures d'incubation de la préparation de viande crue dans le sac d'homogénéisation avec contamination négative (*Staphylococcus aureus* à 4.10⁷ CFU/g) (Figure 6 ligne discontinue) montre une valeur de résistance au transfert d'électron est de 13 kΩ.

Après 6 heures d'enrichissement de la préparation de viande crue avec contamination positive, par *Listeria innocua* ATCC 33090 (0,48 UFC/g de viande crue avant incubation) (figure 6 ligne continue), la valeur de résistance au transfert d'électron est de 45 kΩ, soit environ trois fois supérieure à celle obtenue avec la contamination négative.

Ce résultat montre clairement qu'il est possible de détecter la présence de bactéries *Listeria innocua* présentes dans un échantillon alimentaire avec des fragments Fab' spécifiques fixés sur un biocapteur électrochimique en contact avec un milieu d'enrichissement contenant ledit échantillon sans étape de lavage du capteur, ni amplification du signal.

La valeur R_{ct} moyenne obtenue avec l'intégralité des sacs d'homogénéisation, les valeurs de déviation standard et de coefficient de variation, sont regroupées dans le tableau 2, ci dessous. Les valeurs indiquées sont les valeurs brutes de résistance de charge correspondant au diamètre du demi cercle du signal d'impédance.

**Tableau 2**

| | Contamination negative *S. aureus* (4.10⁷ CFU/g) | Contamination positive *L. innocua* (0,48 UFC/g) |
|---|---|---|
| Moyenne R_{ct} (kΩ) | 15 | 45 |
| Déviation standard | 1 | 8 |
| Coefficient variation (%) | 5 | 17 |
| Nombre de mesures | 16 | 12 |

### Exemple 4 : Elaboration d'un support de capture sensibilisé avec au moins un partenaire de liaison spécifique du microorganisme cible à fins de détection optique

Un support de capture, en polystyrène irradié, commercialisé par la société Nunc/Thermo Scientific (Cat. No. 472230) et représenté sur les figures 9 et 10.

La sensibilisation du support de capture est réalisée, en six étapes, comme suit :
1) le support en polystyrène est immergé à 37°C pendant une nuit dans une solution de BSA (Bovin Serum Albumin)-Biotinylée à 5µg/mL en tampon carbonate pH 9.6 ;
2) le support est ensuite rincé avec un tampon PBS pendant quelques secondes ;
3) après le rinçage, le support est immergé à 37°C pendant deux heures dans une solution de streptavidine à 10µg/mL en tampon phosphate à pH 7.2 ;
4) le support est ensuite rincé avec un tampon carbonate à pH 9.6 pendant quelques secondes ;
5) le support est alors immergé pendant deux heures à 37°C dans une solution de partenaires de liaison spécifiques (1 µg/mL à 40µg/mL) en tampon carbonate à pH 9.6 ;
6) le support est enfin passivé dans une solution de BSA en tampon carbonate à pH 9.6, pendant deux heures à 37°C.

Le support sensibilisé ainsi élaboré peut être utilisé pour la détection optique des microorganismes ou conservé à 2-8°C en vue d'une utilisation ultérieure.

### Exemple 5 : Détection optique de Escherichia coli O157:H7 dans un échantillon alimentaire via l'utilisation d'un support sensibilisé

Le but de cette expérimentation est de détecter directement, via l'utilisation d'un support sensibilisé, tel que décrit *supra* et représenté sur la figure 8, la présence de la bactérie cible *E. coli* O157:H7 dans un échantillon alimentaire en cours d'enrichissement.

Comme détaillé ci-après, la détection s'effectue pendant la période d'incubation en immergeant le support de capture sensibilisé avec une protéine de phage recombinante anti-*E*. *coli* O157:H7 dans un sac d'homogénéisation qui contient l'échantillon alimentaire, dilué au 1/10ème dans le milieu réactionnel.

### Protocole :

### Etape 1 : Remise en suspension des échantillons dans le milieu réactionnel

Quatre échantillons sont préparés comme suit :
- Echantillon A : Dans un sac d'homogénéisation, 25g de steak haché contaminés par 5 unités formant colonies (UFC) de *E. coli* O157:H7 sont remis en suspension dans 225 mL d'EPT (bioMérieux, Réf. 42043) supplémentés par O.Olg/L de vancomycine (Sigma, Cat. No. 75423) et 0.3 g/L de TTC (bioMérieux, Réf. 04568088) ;
- Echantillon B : Dans un sac d'homogénéisation, 25g de steak haché non contaminés par *E. coli* O157:H7 sont remis en suspension dans 225 mL d'EPT supplémentés par O.Olg/L de vancomycine et 0.3 g/L de TTC ;
- Echantillon C : Dans un sac d'homogénéisation, 375g de steak haché contaminés par 5 UFC de *E. coli* O157:H7 sont remis en suspension dans 3375 mL d'EPT supplémentés par O.Olg/L de vancomycine et 0.3 g/L de TTC ;
- Echantillon D : Dans un sac d'homogénéisation, 375g de steak haché non contaminés par *E. coli* O157:H7 sont remis en suspension dans 3375 mL d'EPT supplémentés par O.Olg/L de vancomycine et 0.3 g/L de TTC ;

Pour chaque échantillon, les analyses sont faites en trois exemplaires.

### Etape 2 : Immersion des supports sensibilisés dans les sacs d'homogénéisation avant incubation

Le support de capture sensibilisé est disposé dans chaque sac stomacher (Echantillons. A, B, C et D), comme décrit ci-après. Les sacs d'homogénéisation sont ensuite refermés à l'aide d'une barrette de fermeture et incubés dans une étuve à 41.5°C pendant 16-24h.

### Etape 3 : Lecture des supports de capture à l'issue de la période d'incubation

Au terme de l'incubation (20h à 41.5°C) et suite à la réduction non spécifique du TTC par l'ensemble des bactéries présentes dans l'échantillon (i.e. appartenant à la flore annexe et la flore cible), le milieu réactionnel s'est coloré en rouge. Aussi afin de pouvoir observer le support de capture révélant la positivité ou négativité de l'échantillon analysé, les sacs d'homogénéisation sont inclinés afin d'isoler ledit support de capture du milieu réactionnel.

Conformément au plan expérimental, les échantillons B et D sont positifs alors que les échantillons A et C sont eux négatifs. L'analyse de ces mêmes échantillons par la méthode VIDAS® ECPT, commercialisée par la demanderesse (réf. 30122) a conduit à des résultats analogues, confirmant ainsi les résultats obtenus via lecture optique du support de capture sensibilisé.

Enfin, les niveaux en cible atteints après 20h d'incubation sont de l'ordre de 5.5 log₁₀ UFC/mL pour l'échantillon B et 3.5 log₁₀ UFC/mL pour l'échantillon D.

### Exemple 6 : Détection optique de Listeria spp dans des échantillons environnementaux via l'utilisation d'un support sensibilisé

Le but de cette expérimentation est de détecter directement, via l'utilisation d'un support sensibilisé, la présence de souches bactériennes appartenant au genre *Listeria* dans des échantillons environnementaux en cours d'enrichissement.

Comme détaillé ci-après, la détection s'effectue pendant la période d'incubation en immergeant un support de capture tel que décrit sur la figure 10, sensibilisé avec trois protéines de phage recombinantes anti-*Listeria spp.* dans un conteneur fermé qui contient l'échantillon, dilué dans le milieu réactionnel.

### Protocole :

### Etape 1 : remise en suspension des échantillons dans le milieu réactionnel

L'ensemble des échantillons environnementaux est préparé comme dans l'exemple détaillé ci-après ;
Des éponges (8 cm x 3cm) ayant servi à des prélèvements de surface sont divisées en deux moitiés, traitées comme suit :
Echantillon 1 : Dans un conteneur (i.e ; pilulier), la première 1/2 éponge est contaminée artificiellement par 5 UFC d'une souche appartenant au genre *Listeria* et remise en suspension dans 45 mL de milieu LX (bioMérieux, Réf. 42635) supplémentés par 0.1 g/L de TTC (bioMérieux, Réf. 04568088) ;
Echantillon 2 : Dans un second conteneur (i.e ; pilulier), l'autre moitié non contaminée par une souche appartenant au genre *Listeria* est remise en suspension dans 45 mL de milieu LX (bioMérieux, Réf. 42635) supplémentés par 0.1 g/L de TTC (bioMérieux, Réf.. 04568088).

Le lien entre les échantillons et les souches inoculées artificiellement est présenté dans le tableau 3 ci-dessous :

**Tableau 3**

| **No. Echantillon** | **Souche Inoculée** |
|---|---|
| Echantillon A1 | *L. monocytogenes 4b ATCC 19115* |
| Echantillon A2 | NA |
| Echantillon B1 | *L. seeligeri NSB 22460* |
| Echantillon B2 | NA |
| Echantillon C1 | *L. welshimeri 6a* |
| Echantillon C2 | NA |

### Etape 2 : Immersion du support sensibilisé dans le conteneur (pilulier) avant incubation

Un support sensibilisé, tel que décrit aux figures 9 et 10 est disposé dans chaque pilulier. Pour ce faire, un trou est réalisé dans le couvercle du pilulier de sorte que le support de capture 64 sensibilisé puisse y être rentré en force, jusqu'à ce que la zone d'analyse (zones 641 et 642) soit complètement immergée dans le milieu de culture. Les piluliers sont ensuite refermés avec leur bouchon et incubés dans une étuve à 30°C pendant 24-48h.

### Etape 3 : Lecture des supports de capture à l'issue de la période d'incubation

Au terme de l'incubation (24-48h à 30°C) et suite à la réduction non spécifique du TTC par l'ensemble des bactéries présentes dans l'échantillon (i.e. appartenant à la flore annexe et la flore cible), le milieu de culture s'est coloré en rouge. Aussi afin de pouvoir observer le support de capture sensibilisé révélant la positivité ou négativité de l'échantillon analysé, les sacs d'homogénéisation sont inclinés afin d'isoler ce dernier du milieu réactionnel. Chaque échantillon est également analysé par la méthode VIDAS® LIS (Ref. 30700).

Les résultats obtenus sont répertoriés dans le tableau 4 ci-dessous :

**Tableau 4**

| **No. Echantillon** | **Souche Inoculée** | **Résultat Biocapteur Optique** | **Résultat VIDAS LIS** |
|---|---|---|---|
| *Ech. A1* | *L. monocytogenes 4b ATCC 19115* | + | + |
| *Ech. A2* | NA | - | - |
| *Ech. B1* | *L. seeligeri NSB 22460* | + | + |
| *Ech. B2* | NA | - | - |
| *Ech. C1* | *L. welshimeri 6a* | + | + |
| *Ech. C2* | NA | - | - |

Pour le support de capture sensibilisé, une coloration en rouge de la zone 641 et une absence de coloration de la zone 642 du support de capture mettent en exergue la positivité de l'échantillon (cf. figure 10).

Conformément au plan expérimental, les échantillons 1 sont positifs, alors que les échantillons 2 versus négatifs sont négatifs. L'analyse de ces mêmes échantillons par la méthode VIDAS LIS a conduit à des résultats analogues, confirmant ainsi les résultats obtenus via lecture optique d'un support du capture sensibilisé.

### Exemple 7 : Elaboration des particules fonctionnalisées (conjugués) par au moins un partenaire de liaison spécifique du microorganisme cible

Pour cet exemple, deux types de conjugués sont élaborés à partir de particules de latex d'un diamètre de 400 nm.
- Préparation par adsorption du partenaire de liaison spécifique (protéine de phage recombinante anti-E. coli O157:H7) suivant les étapes comme suit :
   1. lavage des particules de latex (Plain Hidye blue, Polymer lab) en eau VERSOL par centrifugation ;
   2. adsorption des partenaires de liaison spécifiques à 150 µg/mL en tampon phosphate pH 7 en présence des particules de latex à un taux de solide de 0.5% pendant 3 heures à température ambiante et agitation sur roue.

Les rendements d'adsorption sont supérieurs 80%, il n'est donc pas nécessaire d'effectuer un lavage des particules de latex après adsorption.
- Préparation par couplage du partenaire de liaison spécifique (protéine recombinante de phage anti 0157) suivant les étapes comme suit :
   1. lavage des particules de latex (Carboxylique Hidye, Polymer lab) en eau versol par centrifugation ;
   2. couplage à l' éthyl-(N',N'-diméthylamino)propylcarbodiimide hydrochloride (EDC) de la streptavidine à 125 µg/mL en tampon phosphate 20mM pH 7 en présence des particules de latex à un taux de solide de 0.5% par agitation au thermomixer à 37°C et 700 rpm durant 3 heures ;
   3. la streptavidine non couplée est éliminée par centrifugation 20 minutes à 5000g et le culot est repris en tampon Tris 20 mM pH 7 ;
   4. addition du partenaire de liaison spécifique biotinylé (protéine de phage recombinante anti-E. coli O157:H7 biotinylée) à 150 µg/mL et incubation pendant 3 heures à température ambiante et agitation sur roue ;
   5. élimination de l'excès de partenaire de liaison par centrifugation 10 minutes à 7000g et reprise en tampon Tris 20 mM pH 7.

### Exemple 8 : Détection de Escherichia coli O157:H7 dans des échantillons alimentaires via l'agglutination de particules de latex sensibilisées en milieu liquide

Le but de cette expérimentation est de détecter directement, via l'agglutination de particules de latex bleues sensibilisées, tel que décrites à l'exemple précédent, la présence de la bactérie cible E. coli O157:H7 dans un échantillon alimentaire en cours d'enrichissement.

Comme détaillé ci-après, la détection s'effectue pendant la période d'incubation en immergeant les particules de latex bleues sensibilisées avec une protéine de phage recombinante anti-*E*. *coli* O157:H7, dans le conteneur fermé qui contient l'échantillon alimentaire, dilué dans le milieu d'enrichissement.

### Protocole :

### Etape 1 : remise en suspension/dilution des échantillons dans le milieu d'enrichissement

Six échantillons sont préparés comme suit :
Echantillon A1 : Dans un sac d'homogénéisation, 25 mL de lait pasteurisé contaminés par 5 UFC de *E. coli* O157:H7 sont dilués dans 225 mL d'EPT (bioMérieux, réf. 42043) ;
Echantillon A2 : Dans un sac d'homogénéisation, 25 mL de lait pasteurisé non contaminés par *E. coli* O157:H7 sont dilués dans 225 mL d'EPT ;
Echantillon B1 : Dans un sac d'homogénéisation, 25g de saumon contaminés par 5 UFC de *E. coli* O157:H7 sont remis en suspension dans 225 mL d'EPT ;
Echantillon B2 : Dans un sac d'homogénéisation, 25g de saumon non contaminés par *E. coli* O157:H7 sont remis en suspension dans 225 mL d'EPT ;
Echantillon C1 : Dans un sac d'homogénéisation, 25g de salade contaminés par 5 UFC de *E. coli* O157:H7 sont remis en suspension dans 225 mL d'EPT ;
Echantillon C2 : Dans un sac d'homogénéisation, 25g de salade non contaminés par *E. coli* O157:H7 sont remis en suspension dans 225 mL d'EPT ;

Pour chaque échantillon, trois répétitions ont été effectuées.

### Etape 2 : Insertion du tube contenant le milieu réactionnel dans le sac d'homogénéisation avant incubation

Conformément à la figure 11, un tube contenant le milieu réactionnel est ensuite ajouté dans le sac d'homogénéisation. Le milieu réactionnel est composé de 100µl de particules de latex bleues sensibilisées et de 1.4 mL d'EPT supplémenté par 10 mg/L de vancomycine.

Les sacs d'homogénéisation sont ensuite refermés à l'aide d'une barrette de fermeture et placés dans une étuve programmable pour une incubation en trois phases. En effet, le transfert d'un aliquote d'échantillon (0.5 mL) du sac d'homogénéisation vers le tube contenant le milieu réactionnel est réalisé selon le procédé décrit dans le document WO-A-2004/092401, basé sur la loi des gaz parfaits (pV = nRT).

La période d'incubation se décline comme suit :
Phase 1 : 16h à 41.5°C enrichissement des 25g d'échantillon dilués dans l'EPT,
Phase 2 : 1h à 30°C ; transfert de 0.5 mL d'échantillon dans le tube contenant le milieu réactionnel,
Phase 3: 8h à 41.5°C ; enrichissement du milieu réactionnel contenant l' aliquote de 0.5 mL,

Conformément au plan expérimental, les échantillons Ech. No1 ont été déterminés comme positifs versus négatifs pour les échantillons Ech. No2. L'analyse de ces mêmes échantillons par la méthode VIDAS ECPT a conduit à des résultats analogues, confirmant ainsi les résultats obtenus via l'agglutination de particules de latex sensibilisées en milieu liquide.

## Revendications

1. Procédé de détection, d'au moins un microorganisme présent dans un échantillon placé dans un conteneur fermé, ladite méthode comprenant essentiellement les étapes suivantes :
a) Mettre en contact dans le conteneur, ledit échantillon avec
i. au moins un milieu de culture,
ii. un système de révélation apte à permettre la détection
iii. un support apte à capturer le ou les microorganismes à détecter, ledit support étant constitué de particules magnétiques sur lesquelles est fixé au moins un partenaire de liaison spécifique du ou des microorganismes à détecter, ledit partenaire de liaison spécifique étant choisi parmi les anticorps, les fragments Fab, les fragments Fab', les. protéines de phages recombinantes ou non, les phages,
b) Fermer le conteneur,
c) Placer le conteneur dans des conditions aptes à permettre la croissance du ou des microorganisme(s),
d) Appliquer un champ magnétique pour rapprocher les particules magnétiques.
e) Détecter à l'intérieur dudit conteneur fermé, à l'aide d'un moyen de détection, la présence du ou des microorganisme(s) fixé(s) sur le support de capture, la détection étant réalisée en temps réel et mise en évidence par apparition d'une agglutination en temps réel des particules magnétiques.

2. Procédé selon la revendication 1, comportant une étape intermédiaire c') consistant à transférer tout ou partie du mélange constitué par ledit échantillon, le milieu de culture, le support apte à capturer le ou les microorganismes à détecter et éventuellement du système de révélation, du conteneur, dit alors principal vers au moins un deuxième conteneur dit secondaire.

3. Procédé selon l'une des revendications précédentes, comportant une étape supplémentaire f) consistant à confirmer la détection du ou des microorganismes détecté(s).

4. Procédé selon la revendication 3, dans lequel l'étape f) de confirmation est réalisée à l'aide d'un moyen de détection, identique ou différent du moyen de détection utilisé pour l'étape de détection.

5. Procédé de détection selon l'une des revendications précédentes, dans lequel le moyen de détection est pris dans le groupe comprenant : les moyens de détection électriques, notamment électrochimiques, les moyens de détection optiques.

6. Procédé selon l'une des revendications précédentes, le support de capture du ou des microorganisme(s) constitue également le moyen de détection.

7. Procédé de détection selon l'une des revendications précédentes, dans lequel l'application du champ magnétique est répété.

8. Procédé de détection selon l'une des revendications précédentes, dans lequel le conteneur est un sac d'homogénéisation, un flacon, une bouteille, un pilulier.

9. Procédé de détection selon l'une des revendications précédentes, dans lequel le moyen de détection est connecté avec un système d'analyse des données.

10. Procédé de détection selon la revendication 9, dans lequel la connexion entre le support de capture ou le moyen de détection et le dispositif d'analyse des données est une connexion filaire ou une connexion sans fil.

## Patentansprüche

1. Verfahren zum Nachweis mindestens eines Mikroorganismus, der in einer in einem geschlossenen Behälter angeordneten Probe vorhanden ist, wobei das Verfahren im Wesentlichen die folgenden Schritte umfasst:
a) Inkontaktbringen der Probe in dem Behälter mit
i. mindestens einem Kulturmedium,
ii. einem Nachweissystem, das geeignet ist, den Nachweis zu ermöglichen,
iii. einem Träger, der geeignet ist, den oder die nachzuweisenden Mikroorganismen einzufangen, wobei der Träger aus magnetischen Teilchen besteht, an denen mindestens ein spezifischer Verbindungspartner des oder der nachzuweisenden Mikroorganismen fixiert ist, wobei der spezifische Verbindungspartner ausgewählt ist aus den Antikörpern, den Fab-Fragmenten, den Fab'-Fragmenten, den Proteinen von rekombinanten oder nicht rekombinanten Phagen, den Phagen,
b) Schließen des Behälters,
c) Anordnen des Behälters unter Bedingungen, die geeignet sind, das Wachstum des oder der Mikroorganismen zu ermöglichen,
d) Anlegen eines Magnetfeldes, um die magnetischen Teilchen anzunähern,
e) Nachweisen, im Inneren des geschlossenen Behälters mithilfe eines Nachweismittels, des Vorhandenseins des oder der Mikroorganismen, die auf dem Träger zum Einfangen fixiert sind, wobei der Nachweis in Echtzeit durchgeführt wird und durch das Auftreten einer Agglutinierung der magnetischen Teilchen in Echtzeit verdeutlicht wird.

2. Verfahren nach Anspruch 1, welches einen Zwischenschritt c') umfasst, der darin besteht, die Gesamtheit oder einen Teil der Mischung, die aus der Probe, dem Kulturmedium, dem Träger, der geeignet ist, den oder die nachzuweisenden Mikroorganismen einzufangen, und eventuell dem Nachweissystem besteht, von dem Behälter, der dann Hauptbehälter genannt wird, zu mindestens einem zweiten Behälter, Sekundärbehälter genannt, zu übertragen.

3. Verfahren nach einem der vorhergehenden Ansprüche, welches einen zusätzlichen Schritt f) umfasst, der darin besteht, den Nachweis des oder der nachgewiesenen Mikroorganismen zu bestätigen.

4. Verfahren nach Anspruch 3, wobei der Schritt f) der Bestätigung mithilfe eines Nachweismittels durchgeführt wird, das mit dem für den Schritt des Nachweises verwendeten Nachweismittels identisch oder von diesem verschieden ist.

5. Verfahren zum Nachweis nach einem der vorhergehenden Ansprüche, wobei das Nachweismittel aus der Gruppe ausgewählt ist, welche umfasst: die elektrischen, insbesondere elektrochemischen Nachweismittel, die optischen Nachweismittel.

6. Verfahren nach einem der vorhergehenden Ansprüche, wobei der Träger zum Einfangen des oder der Mikroorganismen auch das Nachweismittel darstellt.

7. Verfahren zum Nachweis nach einem der vorhergehenden Ansprüche, wobei das Anlegen des Magnetfeldes wiederholt wird.

8. Verfahren zum Nachweis nach einem der vorhergehenden Ansprüche, wobei der Behälter ein Homogenisierungsbeutel, ein Glasfläschchen, eine Flasche oder eine Pillendose ist.

9. Verfahren zum Nachweis nach einem der vorhergehenden Ansprüche, wobei das Nachweismittel mit einem Datenanalysesystem verbunden ist.

10. Verfahren zum Nachweis nach Anspruch 9, wobei die Verbindung zwischen dem Träger zum Einfangen oder dem Nachweismittel und der Datenanalysevorrichtung eine drahtgebundene Verbindung oder eine drahtlose Verbindung ist.

## Claims

1. A process of detecting at least one microorganism present in a sample placed in a closed container, said method comprising essentially the following steps:
a) Place said sample in contact, in the container, with
i. at least one culture medium
ii a revealing system capable of allowing the detection
iii a support capable of capturing the microorganism(s) to be detected, said support being constituted of magnetic particles onto which is fixed at least one specific binding partner of the microorganism(s) to detect, said specific binding partner is taken from the group comprising : antibodies, Fab fragments, Fab' fragments, recombinant or non-recombinant phage proteins, and phages,
b) Close the container
c) Place the container under conditions capable of allowing the growth of the microorganism(s)
d) Apply a magnetic field for the coming together of the magnetic particles
e) Detect inside said closed container, using detection means, the presence of the microorganism(s) fixed onto the capture support, the detection is performed on real-time and is demonstrated by the appearance of real-time agglutination of the magnetic particles.

2. The process according to claim 1, including an intermediate step c') consisting in transferrring all or part of a mixture constitued by said sample, the culture medium, the support capable of capturing the microorganism(s) to be detected and potentially the reavealing system, from the container, in this case called the main container, to at least one second container called the secondary container.

3. The process according to the one of the preceding claims, including a supplementary step f) consisting in confirming the detection of the microorganism(s) detected.

4. The process according to claim 3, wherein the confirming step f) is accomplished using a detection means which is identical or different from the detection means used for the the detection step.

5. The detection process according to the one of the preceding claims, wherein the detection means is taken from the group comprising: electrical detection means, in particular eletrochemical detection means, optical detection means.

6. The process according to the one of the preceding claims, wherein the support for capturing the microorganism(s) also constitutes the detection means.

7. The detection process according to the one of the preceding claims, wherein the application of the magnetic fied is repeated.

8. The detection process according to the one of the preceding claims, wherein the container is a homogenisation bag, a flask, a bottle or a pillbox.

9. The detection process according to the one of the preceding claims, wherein the detection means is connected to a data analysis system.

10. The detection process according to claim 9 wherein the connection between the capture support or the detection means and the data analysis device is a wired connection or a wireless connection.
